Europäisches Patentamt

European Patent Office

Office européen des brevets

(11) Publication number: **0 257 630**
**A1**

## (12) EUROPEAN PATENT APPLICATION

(21) Application number: 87112381.6

(22) Date of filing: 26.08.87

(51) Int. Cl.⁴: **C12N 15/00** , C07H 21/04 , C12P 21/00 , C07K 13/00

The applicant has filed a statement in accordance with Rule 28 (4) EPC (issue of a sample only to an expert). Accession number(s) of the deposit(s): FERM BP-1350.

(30) Priority: 27.08.86 JP 199056/86
05.12.86 JP 288875/86

(43) Date of publication of application:
02.03.88 Bulletin 88/09

(84) Designated Contracting States:
AT BE CH DE ES FR GB GR IT LI LU NL SE

(71) Applicant: HOECHST JAPAN LIMITED
10-16, 8-chome, Akasaka, Minato-ku
Tokyo(JP)

(72) Inventor: Tone, Masahide
Sekine Apartment A-201 345-2 Kitairiso
Sayama-Shi Saitama-Ken(JP)
Inventor: Iwaki, Akiko
275 Hoym St., No. 4B
Fort Lee, N.J. 07024(US)
Inventor: Kikuno, Reiko
Villa Charman 102 561-5 Kume
Tokorozawa-Shi Saitama-Ken(JP)
Inventor: Hashimoto, Tamotsu
1-5-10-309, Sakae-Cho
Asaka-Shi Saitama-Ken(JP)
Inventor: Okazaki, Hiroshi
3-9-11-402, Irima-Cho
Chofu-Shi Tokyo(JP)

(74) Representative: Meyer-Dulheuer,
Karl-Hermann, Dr. et al
HOECHST Aktiengesellschaft Zentrale
Patentabteilung Postfach 80 03 20
D-6230 Frankfurt/Main 80(DE)

(54) Alpha2-Plasmin inhibitor-like protein-coding DNA.

(57) DNA having mRNA-derived genetic information that encodes $\alpha_2$-plasmin inhibitor-like protein. The DNA can be used in preparing $\alpha_2$-plasmin inhibitor-like protein by integrating into a plasmin vector containing an expression promoter to express in a host such as animals cells.

## 0 257 630

### α₂-Plasmin inhibitor-like protein-coding DNA

Background of the Invention

1. Field of the Invention

The present invention relates to a novel DNA which has mRNA-derived genetic information that codes $\alpha_2$-plasmin inhibitor-like protein.

The DNA of the invention can be used to produce $\alpha_2$-plasmin inhibitor-like protein by such a process as integrating into a plasmid vector containing an expression promoter in the form of a cDNA shown in Table 2 described below to express in a host such as animal cells.

2. Description of Prior Arts

There are known in human plasma such plasmin-antagonizing substances as $\alpha_2$-macroglobulin, $\alpha_1$-macroglobulin, $\alpha_1$-antitrypsin, $\alpha_2$-plasmin inhibitor and antithrombin III. Among them, the $\alpha_2$-plasmin inhibitor is a very important substance due to its highest affinity to plasmin, though it exists in human plasma in a small quantity. Fibrin formed during blood coagulation process is decomposed by the action of plasmin, and plasmin inhibitor antagonizes the action of plasmin by binding with it. If the plasmin inhibitor is supplied in a large amount at a lower price, it will be useful as an antihemorrhagic agent or a composent of the tissue adhesive.

$\alpha_2$-Plasmin inhibitor is a single-stranded glycoprotein with a molecular weight of 65,000-70,000 and is contained in human plasma in a concentration as low as 0.7-1 $\mu M$. Heretofore, it has not been isolated and clinically applied.

Amino acid sequence of the $\alpha_2$-plasmin inhibitor has partially been elucidated by H.R. Lijnen et al. (Literature and other references described below).

Further, Fair, D. S. et al. have elucidated that a small amount of $\alpha_2$-plasmin inhibitor is produced in a supernatant of a cultured medium of the cells named Hep G2, which is one of the cell lines of Human hepatic cell cancer (J. Lab. Clin. Med., 101 , P. 372 (1983)).

W. E. Holmes et al. in Journal Cell Biochem. Suppl. 10A, 274 report that a cDNA clone of $\alpha_2$-plasmin inhibitor was obtained by using human liver as a source of mRNA, and that amino acid sequence of the $\alpha_2$-plasmin inhibitor deduced from the cDNA was compared with amino acid sequences of $\alpha_2$-plasmin inhibitor and related substances. There is, however, no particular description of base sequence of the cDNA and the entire amino acid sequence of $\alpha_2$-plasmin inhibitor deduced from it.

Although $\alpha_2$-plasmin inhibitor could be used as an antihemorrhagic agent or a component of the tissue adhesive as mentioned above, it is not commercially available because of its very small quantity in plasma.

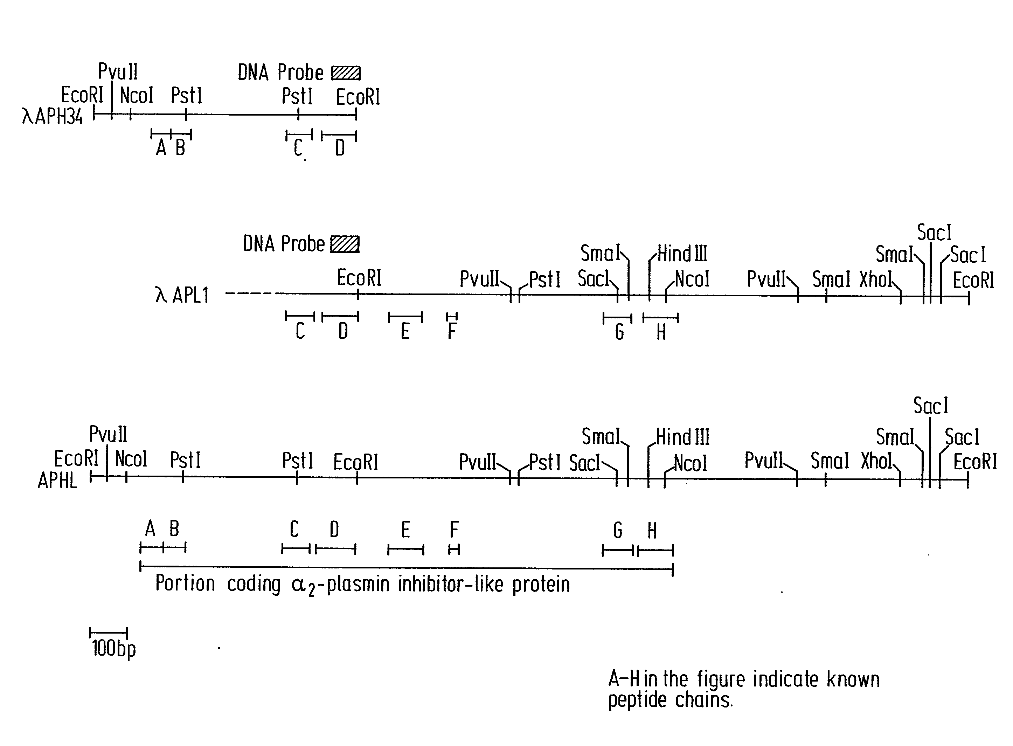

Brief Description of the Drawing

The attached drawing shows the restriction enzyme map of APHL of the Invention.

Summary of the Invention

As a result of extensive studies to provide $\alpha_2$-plasmin inhibitor or a physiologically similar substance thereto, we have completed the present invention.

According to the invention, there is provided the following novel DNA:

1) DNA coding human $\alpha_2$-plasmin inhibitor-like protein of the amino acid sequence.

Asn-Gln-Glu-Gln-Val-Ser-Pro-Leu-Thr-

Leu-Leu-Lys-Leu-Gly-Asn-Gln-Glu-Pro-Gly-

Gly-Gln-Thr-Ala-Leu-Lys-Ser-Pro-Pro-Gly-

Val-Cys-Ser-Arg-Asp-Pro-Thr-Pro-Glu-Gln-

Thr-His-Arg-Leu-Ala-Arg-Ala-Met-Met-Ala-

Phe-Thr-Ala-Asp-Leu-Phe-Ser-Leu-Val-Ala-

Gln-Thr-Ser-Thr-Cys-Pro-Asn-Leu-Ile-Leu-

Ser-Pro-Leu-Ser-Val-Ala-Leu-Ala-Leu-Ser-

His-Leu-Ala-Leu-Gly-Ala-Gln-Asn-His-Thr-

Leu-Gln-Arg-Leu-Gln-Gln-Val-Leu-His-Ala-

Gly-Ser-Gly-Pro-Cys-Leu-Pro-His-Leu-Leu-

Ser-Arg-Leu-Cys-Gln-Asp-Leu-Gly-Pro-Gly-

Ala-Phe-Arg-Leu-Ala-Ala-Arg-Met-Tyr-Leu-

Gln-Lys-Gly-Phe-Pro-Ile-Lys-Glu-Asp-Phe-

Leu-Glu-Gln-Ser-Glu-Gln-Leu-Phe-Gly-Ala-

Lys-Pro-Val-Ser-Leu-Thr-Gly-Lys-Gln-Glu-

Asp-Asp-Leu-Ala-Asn-Ile-Asn-Gln-Trp-Val-

Lys-Glu-Ala-Thr-Glu-Gly-Lys-Ile-Gln-Glu-

Phe-Leu-Ser-Gly-Leu-Pro-Glu-Asp-Thr-Val-

Leu-Leu-Leu-Leu-Asn-Ala-Ile-His-Phe-Gln-

Gly-Phe-Trp-Arg-Asn-Lys-Phe-Asp-Pro-Ser-

Leu-Thr-Gln-Arg-Asp-Ser-Phe-His-Leu-Asp-

Glu-Gln-Phe-Thr-Val-Pro-Val-Glu-Met-Met-

Gln-Ala-Arg-Thr-Tyr-Pro-Leu-Arg-Trp-Phe-

Leu-Leu-Glu-Gln-Pro-Glu-Ile-Gln-Val-Ala-

His-Phe-Pro-Phe-Lys-Asn-Asn-Met-Ser-Phe-

Val-Val-Leu-Val-Pro-Thr-His-Phe-Glu-Trp-

Asn-Val-Ser-Gln-Val-Leu-Ala-Asn-Leu-Ser-

Trp-Asp-Thr-Leu-His-Pro-Pro-Leu-Val-Trp-

Glu-Arg-Pro-Thr-Lys-Val-Arg-Leu-Pro-Lys-

Leu-Tyr-Leu-Lys-His-Gln-Met-Asp-Leu-Val-

Ala-Thr-Leu-Ser-Gln-Leu-Gly-Leu-Gln-Glu-

Leu-Phe-Gln-Ala-Pro-Asp-Leu-Arg-Gly-Ile-

Ser-Glu-Gln-Ser-Leu-Val-Val-Ser-Gly-Val-

Gln-His-Gln-Ser-Thr-Leu-Glu-Leu-Ser-Glu-

Val-Gly-Val-Glu-Ala-Ala-Ala-Ala-Thr-Ser-

Ile-Ala-Met-Ser-Arg-Met-Ser-Leu-Ser-Ser-

Phe-Ser-Val-Asn-Arg-Pro-Phe-Leu-Phe-Phe-

Ile-Phe-Glu-Asp-Thr-Thr-Gly-Leu-Pro-Leu-

Phe-Val-Gly-Ser-Val-Arg-Asn-Pro-Asn-Pro-

Ser-Ala-Pro-Arg-Glu-Leu-Lys-Glu-Gln-Gln-

Asp-Ser-Pro-Gly-Asn-Lys-Asp-Phe-Leu-Gln-

Ser-Leu-Lys-Gly-Phe-Pro-Arg-Gly-Asp-Lys-

Leu-Phe-Gly-Pro-Asp-Leu-Lys-Leu-Val-Pro-

Pro-Met-Glu-Glu-Asp-Tyr-Pro-Gln-Phe-Gly-

Ser-Pro-Lys.

2) DNA according to the above item 1) having the base sequence,

```
AAC.CAG.GAG.CAG.GTG.TCC.CCA.CTT.ACC.

CTC.CTC.AAG.TTG.GGC.AAC.CAG.GAG.CCT.GGT.

GGC.CAG.ACT.GCC.CTG.AAG.AGT.CCC.CCA.GGA.

GTC.TGC.AGC.AGA.GAC.CCC.ACC.CCA.GAG.CAG.

ACC.CAC.AGG.CTG.GCC.CGG.GCC.ATG.ATG.GCC.

TTC.ACT.GCC.GAC.CTG.TTC.TCC.CTG.GTG.GCT.

CAA.ACG.TCC.ACC.TGC.CCC.AAC.CTC.ATC.CTG.

TCA.CCC.CTG.AGT.GTG.GCC.CTG.GCG.CTG.TCT.

CAC.CTG.GCA.CTA.GGT.GCT.CAG.AAC.CAC.ACG.

TTG.CAG.AGG.CTG.CAA.CAG.GTG.CTG.CAC.GCA.

GGC.TCA.GGG.CCC.TGC.CTC.CCC.CAT.CTG.CTG.

AGC.CGC.CTC.TGC.CAG.GAC.CTG.GGC.CCC.GGC.

GCG.TTC.CGA.CTG.GCT.GCC.AGG.ATG.TAC.CTG.

CAG.AAA.GGA.TTT.CCC.ATC.AAA.GAA.GAT.TTC.

CTG.GAA.CAA.TCC.GAA.CAG.CTA.TTT.GGG.GCA.

AAG.CCC.GTG.AGC.CTG.ACG.GGA.AAG.CAG.GAA.

GAT.GAC.CTG.GCA.AAC.ATC.AAC.CAA.TGG.GTG.

AAG.GAG.GCC.ACG.GAG.GGG.AAG.ATT.CAG.GAA.

TTC.CTC.TCT.GGG.CTG.CCG.GAA.GAC.ACC.GTG.
```

TTG.CTT.CTC.CTC.AAC.GCC.ATC.CAC.TTC.CAG.

GGT.TTC.TGG.AGG.AAC.AAG.TTT.GAC.CCG.AGC.

CTT.ACC.CAG.AGA.GAC.TCC.TTC.CAC.CTG.GAC.

GAG.CAG.TTC.ACG.GTG.CCC.GTG.GAA.ATG.ATG.

CAG.GCC.CGC.ACG.TAC.CCG.CTG.CGC.TGG.TTC.

TTG.CTG.GAG.CAG.CCT.GAG.ATC.CAG.GTG.GCT.

CAT.TTC.CCC.TTT.AAG.AAC.AAC.ATG.AGC.TTT.

GTG.GTC.CTT.GTA.CCC.ACC.CAC.TTT.GAA.TGG.

AAC.GTG.TCC.CAG.GTA.CTG.GCC.AAC.CTG.AGT.

TGG.GAC.ACC.CTG.CAC.CCA.CCT.CTG.GTG.TGG.

GAG.AGG.CCC.ACC.AAG.GTC.CGG.CTG.CCT.AAG.

CTG.TAT.CTG.AAA.CAC.CAA.ATG.GAC.CTG.GTG.

GCC.ACC.CTC.AGC.CAG.CTG.GGC.CTG.CAG.GAG.

TTG.TTC.CAG.GCC.CCA.GAC.CTG.CGT.GGG.ATC.

TCC.GAG.CAG.AGC.CTG.GTG.GTG.TCC.GGC.GTG.

CAG.CAT.CAG.TCC.ACC.CTG.GAG.CTC.AGC.GAG.

GTC.GGC.GTG.GAG.GCG.GCG.GCG.GCC.ACC.AGC.

ATT.GCC.ATG.TCC.CGC.ATG.TCC.CTG.TCC.TCC.

TTC.AGC.GTG.AAC.CGC.CCC.TTC.CTC.TTC.TTC.

ATC.TTC.GAG.GAC.ACC.ACA.GGC.CTT.CCC.CTC.

```
TTC.GTG.GGC.AGC.GTG.AGG.AAC.CCC.AAC.CCC.

AGT.GCA.CCG.CGG.GAG.CTC.AAG.GAA.CAG.CAG.

GAT.TCC.CCG.GGC.AAC.AAG.GAC.TTC.CTC.CAG.

AGC.CTG.AAA.GGC.TTC.CCC.CGC.GGA.GAC.AAG.

CTT.TTC.GGC.CCT.GAC.TTA.AAA.CTT.GTG.CCC.

CCC.ATG.GAG.GAG.GAT.TAC.CCC.CAG.TTT.GGC.

AGC.CCC.AAG,
```

The $\alpha_2$-plasmin inhibitor-like protein encoded in the DNA of the invention is composed of the 452 amino acids shown by the above-mentioned sequence which is identical with $\alpha_2$-plasmin inhibitor in major amino acid-sequence moiety. The amino acid sequence of the $\alpha_2$-plasmin inhibitor-like protein identified in the present invention, however, is different partly from the partial sequence of $\alpha_2$-plasmin inhibitor described in the literatures in the following aspects.

(1) Whereas the protein encoded in the DNA of the invention contains only 4 cystines, there is reported in the literature (Reference 1) that 6 cystines are contained.

(2) There are differences in at least 7 amino acid residues.

(3) Whereas the carboxyl end group of human $\alpha_2$-plasmin inhibitor is reported to be the amino acid sequence of phenylalanine-leucine (H.R. Lijnen et al., Thromb Haemostas (Stuttgart) 48 (3), 311-314 (1982)) the carboxyl end group of the protein encoded in the DNA of the invention is proline-lysine.

However, as the amino acid sequence is in major portions identical with that reported in the literatures the protein encoded in the DNA of the invention is believed to have at least a structure similar to the structure of human $\alpha_2$-plasmin inhibitor.

In addition, there are some sequences in the deduced amino acid sequence in the invention at which a glycosylation can occur such as Asn-X-Ser, Asn-X-Thr, thereby suggesting that saccharides are bonded in such portions.

First, m-RNAs were isolated from Hep G2 cells (ATCC HB8065) which has been known to produce $\alpha_2$-plasmin inhibitor. A cDNA library was prepared by using the m-RNAs as templates. The above-mentioned cDNA library was then screened using a probe (Table 1) consisting of a DNA that encodes a portion of the amino acid sequence of $\alpha_2$-plasmin inhibitor which was partially elucidated by H.R. Lijner et al. to give a cDNA clone named λAPH34 that formed a hybrid with said probe.

Separately, a human liver cDNA library commercially available from Toyo Boseki K.K. was screened employing the same probe to give a cDNA clone named λAPL1 that contained a cDNA insert fragment hybridizing to the probe.

After determination of the DNA sequences in the portion and neighborhood thereof where the two clones hybridized to the probe, the cDNA fragments derived from the two clones were concluded to be in such a relation as indicated in the drawing, thereby producing a complete cDNA strand APHL.

Base sequence of the cDNA strand APHL is shown in Table 2.

0 257 630

## Table 1

### Preparation of the DNA probe

Gln-Glu-Asp-Asp-Leu-Ala-Asn-Ile-Asn-Gln-Trp-Val-Lys-Glu-Ala-Thr-Glu-Gly-Lys-Ile-Gln-Glu-Phe

```
5'-G GAG GAC GAC CTG GCC AAC ATC AAC CAG TGG GTG AAG GAG GCC ACC GAG GGC AAG ATC CAG G-3'
3'-ACGTC CTC CTG CTG GAC CGG TTG TAG TTG GTC ACC CAC TTC CTC CGG TGG CTC CCG TTC TAG GTC CTT AA-5'} → Probe
                                                                                                      portion
```

Pst I                                                                        EcoR I

## Table 2

```
                                                                    24*
                                        GAA.TTC.CGG.CTG.GCA.GGG.GAG.AAC

*  25                                                               84*
ATG.GCG.CTG.CTC.TGG.GGG.CTC.CTG.GTG.CTC.AGC.TGG.TCC.TGC.CTG.CAA.GGC.CCC.TGC.TCC
Met-Ala-Leu-Leu-Trp-Gly-Leu-Leu-Val-Leu-Ser-Trp-Ser-Cys-Leu-Gln-Gly-Pro-Cys-Ser

*  85                                                              144*
GTG.TTC.TCC.CCT.GTG.AGC.GCC.ATG.GAG.CCC.TTG.GGC.TGG.CAG.CTA.ACT.AGC.GGG.CCG.AAC
Val-Phe-Ser-Pro-Val-Ser-Ala-Met-Glu-Pro-Leu-Gly-Trp-Gln-Leu-Thr-Ser-Gly-Pro-Asn

*  145                                                             204*
CAG.GAG.CAG.GTG.TCC.CCA.CTT.ACC.CTC.CTC.AAG.TTG.GGC.AAC.CAG.GAG.CCT.GGT.GGC.CAG
Gln-Glu-Gln-Val-Ser-Pro-Leu-Thr-Leu-Leu-Lys-Leu-Gly-Asn-Gln-Glu-Pro-Gly-Gly-Gln

*  205                                                             264*
ACT.GCC.CTG.AAG.AGT.CCC.CCA.GGA.GTC.TGC.AGC.AGA.GAC.CCC.ACC.CCA.GAG.CAG.ACC.CAC
Thr-Ala-Leu-Lys-Ser-Pro-Pro-Gly-Val-Cys-Ser-Arg-Asp-Pro-Thr-Pro-Glu-Gln-Thr-His

*  265                                                             324*
AGG.CTG.GCC.CGG.GCC.ATG.ATG.GCC.TTC.ACT.GCC.GAC.CTG.TTC.TCC.CTG.GTG.GCT.CAA.ACG
Arg-Leu-Ala-Arg-Ala-Met-Met-Ala-Phe-Thr-Ala-Asp-Leu-Phe-Ser-Leu-Val-Ala-Gln-Thr

*  325                                                             384*
TCC.ACC.TGC.CCC.AAC.CTC.ATC.CTG.TCA.CCC.CTG.AGT.GTG.GCC.CTG.GCG.CTG.TCT.CAC.CTG
Ser-Thr-Cys-Pro-Asn-Leu-Ile-Leu-Ser-Pro-Leu-Ser-Val-Ala-Leu-Ala-Leu-Ser-His-Leu

*  385                                                             444*
GCA.CTA.GGT.GCT.CAG.AAC.CAC.ACG.TTG.CAG.AGG.CTG.CAA.CAG.GTG.CTG.CAC.GCA.GGC.TCA
Ala-Leu-Gly-Ala-Gln-Asn-His-Thr-Leu-Gln-Arg-Leu-Gln-Gln-Val-Leu-His-Ala-Gly-Ser

*  445                                                             504*
GGG.CCC.TGC.CTC.CCC.CAT.CTG.CTG.AGC.CGC.CTC.TGC.CAG.GAC.CTG.GGC.CCC.GGC.GCG.TTC
Gly-Pro-Cys-Leu-Pro-His-Leu-Leu-Ser-Arg-Leu-Cys-Gln-Asp-Leu-Gly-Pro-Gly-Ala-Phe

*  505                                                             564*
CGA.CTG.GCT.GCC.AGG.ATG.TAC.CTG.CAG.AAA.GGA.TTT.CCC.ATC.AAA.GAA.GAT.TTC.CTG.GAA
Arg-Leu-Ala-Ala-Arg-Met-Tyr-Leu-Gln-Lys-Gly-Phe-Pro-Ile-Lys-Glu-Asp-Phe-Leu-Glu

*  565                                                             624*
CAA.TCC.GAA.CAG.CTA.TTT.GGG.GCA.AAG.CCC.GTG.AGC.CTG.ACG.GGA.AAG.CAG.GAA.GAT.GAC
Gln-Ser-Glu-Gln-Leu-Phe-Gly-Ala-Lys-Pro-Val-Ser-Leu-Thr-Gly-Lys-Gln-Glu-Asp-Asp

*  625                                                             684*
CTG.GCA.AAC.ATC.AAC.CAA.TGG.GTG.AAG.GAG.GCC.ACG.GAG.GGG.AAG.ATT.CAG.GAA.TTC.CTC
Leu-Ala-Asn-Ile-Asn-Gln-Trp-Val-Lys-Glu-Ala-Thr-Glu-Gly-Lys-Ile-Gln-Glu-Phe-Leu

*  685                                                             744*
TCT.GGG.CTG.CCG.GAA.GAC.ACC.GTG.TTG.CTT.CTC.CTC.AAC.GCC.ATC.CAC.TTC.CAG.GGT.TTC
Ser-Gly-Leu-Pro-Glu-Asp-Thr-Val-Leu-Leu-Leu-Leu-Asn-Ala-Ile-His-Phe-Gln-Gly-Phe

*  745                                                             804*
TGG.AGG.AAC.AAG.TTT.GAC.CCG.AGC.CTT.ACC.CAG.AGA.GAC.TCC.TTC.CAC.CTG.GAC.GAG.CAG
Trp-Arg-Asn-Lys-Phe-Asp-Pro-Ser-Leu-Thr-Gln-Arg-Asp-Ser-Phe-His-Leu-Asp-Glu-Gln
```

```
*  805                                                                    864*
TTC.ACG.GTG.CCC.GTG.GAA.ATG.ATG.CAG.GCC.CGC.ACG.TAC.CCG.CTG.CGC.TGG.TTC.TTG.CTG
Phe-Thr-Val-Pro-Val-Glu-Met-Met-Gln-Ala-Arg-Thr-Tyr-Pro-Leu-Arg-Trp-Phe-Leu-Leu

*  865                                                                    924*
GAG.CAG.CCT.GAG.ATC.CAG.GTG.GCT.CAT.TTC.CCC.TTT.AAG.AAC.AAC.ATG.AGC.TTT.GTG.GTC
Glu-Gln-Pro-Glu-Ile-Gln-Val-Ala-His-Phe-Pro-Phe-Lys-Asn-Asn-Met-Ser-Phe-Val-Val

*  925                                                                    984*
CTT.GTA.CCC.ACC.CAC.TTT.GAA.TGG.AAC.GTG.TCC.CAG.GTA.CTG.GCC.AAC.CTG.AGT.TGG.GAC
Leu-Val-Pro-Thr-His-Phe-Glu-Trp-Asn-Val-Ser-Gln-Val-Leu-Ala-Asn-Leu-Ser-Trp-Asp

*  985                                                                   1044*
ACC.CTG.CAC.CCA.CCT.CTG.GTG.TGG.GAG.AGG.CCC.ACC.AAG.GTC.CGG.CTG.CCT.AAG.CTG.TAT
Thr-Leu-His-Pro-Pro-Leu-Val-Trp-Glu-Arg-Pro-Thr-Lys-Val-Arg-Leu-Pro-Lys-Leu-Tyr

* 1045                                                                   1104*
GTG.AAA.CAC.CAA.ATG.GAC.CTG.GTG.GCC.ACC.CTC.AGC.CAG.CTG.GGC.CTG.CAG.GAG.TTG.TTC
Leu-Lys-His-Gln-Met-Asp-Leu-Val-Ala-Thr-Leu-Ser-Gln-Leu-Gly-Leu-Gln-Glu-Leu-Phe

* 1105                                                                   1164*
CAG.GCC.CCA.GAC.CTG.CGT.GGG.ATC.TCC.GAG.CAG.AGC.CTG.GTG.GTG.TCC.GGC.GTG.CAG.CAT
Gln-Ala-Pro-Asp-Leu-Arg-Gly-Ile-Ser-Glu-Gln-Ser-Leu-Val-Val-Ser-Gly-Val-Gln-His

* 1165                                                                   1224*
CAG.TCC.ACC.CTG.GAG.CTC.AGC.GAG.GTC.GGC.GTG.GAG.GCG.GCG.GCG.GCC.ACC.AGC.ATT.GCC
Gln-Ser-Thr-Leu-Glu-Leu-Ser-Glu-Val-Gly-Val-Glu-Ala-Ala-Ala-Ala-Thr-Ser-Ile-Ala

* 1225                                                                   1284*
ATG.TCC.CGC.ATG.TCC.CTG.TCC.TCC.TTC.AGC.GTG.AAC.CGC.CCC.TTC.CTC.TTC.TTC.ATC.TTC
Met-Ser-Arg-Met-Ser-Leu-Ser-Ser-Phe-Ser-Val-Asn-Arg-Pro-Phe-Leu-Phe-Phe-Ile-Phe

* 1285                                                                   1344*
GAG.GAC.ACC.ACA.GGC.CTT.CCC.CTC.TTC.GTG.GGC.AGC.GTG.AGG.AAC.CCC.AAC.CCC.AGT.GCA
Glu-Asp-Thr-Thr-Gly-Leu-Pro-Leu-Phe-Val-Gly-Ser-Val-Arg-Asn-Pro-Asn-Pro-Ser-Ala

* 1345                                                                   1404*
CCG.CGG.GAG.CTC.AAG.GAA.CAG.CAG.GAT.TCC.CCG.GGC.AAC.AAG.GAC.TTC.CTC.CAG.AGC.CTG
Pro-Arg-Glu-Leu-Lys-Glu-Gln-Gln-Asp-Ser-Pro-Gly-Asn-Lys-Asp-Phe-Leu-Gln-Ser-Leu

* 1405                                                                   1464*
AAA.GGC.TTC.CCC.CGC.GGA.GAC.AAG.CTT.TTC.GGC.CCT.GAC.TTA.AAA.CTT.GTG.CCC.CCC.ATG
Lys-Gly-Phe-Pro-Arg-Gly-Asp-Lys-Leu-Phe-Gly-Pro-Asp-Leu-Lys-Leu-Val-Pro-Pro-Met

* 1465                                                                   1524*
GAG.GAG.GAT.TAC.CCC.CAG.TTT.GGC.AGC.CCC.AAG.TGA.GGG.GCC.GTG.GCT.GTG.GCA.TCC.AGA
Glu-Glu-Asp-Tyr-Pro-Gln-Phe-Gly-Ser-Pro-Lys-***-Gly-Ala-Val-Ala-Val-Ala-Ser-Arg

* 1525                                                                   1584*
GTC.CCT.GCC.TGG.ACC.AGC.CTC.TCC.ACT.CAT.GTG.ACT.CTT.TCC.AAC.CTG.CTT.TGT.GGC.ACT
Val-Pro-Ala-Trp-Thr-Ser-Leu-Ser-Thr-His-Val-Thr-Leu-Ser-Asn-Leu-Leu-Cys-Gly-Thr

* 1585                                                                   1644*
GGG.GCA.GGG.GCC.GGG.GGC.AGT.CTG.AGA.GAG.GCC.ATT.CTT.TCC.CAA.CAC.CTC.TTG.GGG.AGT
Gly-Ala-Gly-Ala-Gly-Gly-Ser-Leu-Arg-Glu-Ala-Ile-Leu-Ser-Gln-His-Leu-Leu-Gly-Ser
```

11

```
* 1645                                                                      1704*
TTA.GGG.TGG.GGG.GGG.GCG.CGG.CTG.GGA.GGA.GGG.CAG.GCA.TCG.GGG.AGC.CGG.GAG.CCT.GAC
Leu-Gly-Trp-Gly-Gly-Ala-Arg-Leu-Gly-Gly-Gly-Gln-Ala-Ser-Gly-Ser-Arg-Glu-Pro-Asp

* 1705                                                                      1764*
CCT.CAT.CTT.TCT.TCC.AAA.CAG.GCT.CAG.AGG.GTG.TCC.TGC.ACC.GGG.GCC.TGG.GCA.GGA.GGG
Pro-His-Leu-Ser-Ser-Lys-Gln-Ala-Gln-Arg-Val-Ser-Cys-Thr-Gly-Ala-Trp-Ala-Gly-Gly

* 1765                                                                      1824*
AGG.TGC.TTC.TAG.TTC.TGC.CAG.GAG.ACA.GGT.TAG.CTG.CTC.CCC.ACG.TCA.GCT.GGG.ACA.CCC
Arg-Cys-Phe-***-Phe-Cys-Gln-Glu-Thr-Gly-***-Leu-Leu-Pro-Thr-Ser-Ala-Gly-Thr-Pro

* 1825                                                                      1884*
CGA.CTT.TTG.TTT.ACC.AGA.GAA.AAA.GGG.AGG.GGG.AGA.GGG.CTG.CCT.TTG.GAC.TTG.TCC.CGG
Arg-Leu-Leu-Phe-Thr-Arg-Glu-Lys-Gly-Arg-Gly-Arg-Gly-Leu-Pro-Leu-Asp-Leu-Ser-Arg

* 1885                                                                      1944*
GAC.ACC.TAG.GCT.AGG.GTG.GGG.AGA.GAC.GGG.CCC.TGG.TGG.TGG.CTC.GGG.AGG.CGA.ACG.TTG
Asp-Thr-***-Ala-Arg-Val-Gly-Arg-Asp-Gly-Pro-Trp-Trp-Trp-Leu-Gly-Arg-Arg-Thr-Leu

* 1945              .                                                       2004*
TCC.TCA.GCC.CCG.CGT.GGA.ACT.CGT.GTC.TGG.CAC.AGC.CTG.GCT.GTG.GCC.TAA.CCT.GCC.GAG
Ser-Ser-Ala-Pro-Arg-Gly-Thr-Arg-Val-Trp-His-Ser-Leu-Ala-Val-Ala-***-Pro-Ala-Glu

* 2005                                                                      2064*
AGT.CCA.TCA.GCC.TCC.ATC.CTA.CCC.CCT.GTG.CCT.TGT.CAC.GCC.AGA.CTT.CCC.ACG.GCT.CCT
Ser-Pro-Ser-Ala-Ser-Ile-Leu-Pro-Pro-Val-Pro-Cys-His-Ala-Arg-Leu-Pro-Thr-Ala-Pro

* 2065                                                                      2124*
CGA.GAT.CCC.AAC.ACT.GCC.AGC.ATT.TCC.CTT.CCT.TCC.TCT.CCT.GTC.TCC.CTC.CTC.TGC.CCG
Arg-Asp-Pro-Asn-Thr-Ala-Ser-Ile-Ser-Leu-Pro-Ser-Ser-Pro-Val-Ser-Leu-Leu-Cys-Pro

* 2125                                                                      2184*
GGA.GCT.CAG.GAA.CCG.AGG.CAG.GGA.AGG.ATC.CCA.TGA.GCT.CCT.TAA.GGC.TCT.TTT.GTA.AGG
Gly-Ala-Gln-Glu-Pro-Arg-Gln-Gly-Arg-Ile-Pro-***-Ala-Pro-***-Gly-Ser-Phe-Val-Arg

* 2185                                                                      2244*
TTT.TTG.TAG.TGA.TTT.TTA.TGC.CAC.CTG.AAT.AAA.GAA.TGA.ATG.GGC.AAA.AAA.AAA.AAA.AAA
Phe-Leu-***-***-Phe-Leu-Cys-His-Leu-Asn-Lys-Glu-***-Met-Gly-Lys-Lys-Lys-Lys-Lys

* 2245
AAA.AAA.GGA.ATT.
Lys-Lys-Gly-Ile
```

### Number in the table is the number of the base marked *.

In the base sequence, the nucleotide positions from 1 to 6 represent the EcoRI site derived from the EcoRI linker used in the cloning, and those from 7 to 24 represent the 5′-noncoding area. Those from 25 to 141 represent the peptide portion observed in the $\alpha_2$-plasmin inhibitor precurser only (so-called the anchor portion or leader sequence portion). The sequence between positions 142 and 1497 is the portion that encodes the desired $\alpha_2$-plasmin inhibitor-like substance. TGA sequence at positions 1498 to 1500 is the stop codon. The sequence after position 1501 is the 3'-noncoding area, the sequence of AATAAA at positions 2212 to 2217 is the poly A addition signal and the poly A sequence after 2250 is the area derived from the poly A end which normally exists in messenger RNA. The nucleotide sequence between 2251 and 2256 is the EcoRI site derived from the EcoRI linker employed for the cloning.

The DNA of the invention can be prepared, for example, by the method as described below.

Hep G2 cell line was cultivated according to a conventional method, and from the cultured cells was separated by the guanidium isocyanate/cesium chloride method the whole RNA from which a poly A messenger RNA was isolated by the oligo dT column chromatography. A cDNA library was constructed which is complementary to the poly A messenger RNA by the method employing λgt11, transfected into E. coli (Y1090) and then it was cultivated.

There was prepared a synthetic probe described in Table 1 which is complementary to the DNA sequence corresponding to the amino acid sequence of Table 1 described by H.R. Lijnen et al. in Thromb Haemostas (Stuttgart) 48 (3), 311-314 (1982) as a partial amino acid sequence of $\alpha_2$-plasmin inhibitor. Positive plaques were selected by using the probe. The plaques were cultivated, treated in a conventional manner and phage genomes were then digested with EcoRI to produce the cDNA fragment λAPH34.

Separately, a human liver cDNA library commercially available from Toyo Boseki K.K. which was called Human Liver cDNA ( λ gt11) was screened using the probe as in Table 1, and the resulting phage genome DNA (λAPL1) was digested with EcoRI. The desired cDNA fragment APHL was made by fusing the two cDNA fragments (λAPH34 and λAPL1) obtained. Restriction enzyme map of the APHL is set forth in the attached drawing.

The present invention will be described in more details by the following examples:

Example 1

Isolation of a messenger RNA

Hep G2 cell strain was received from the American Type Culture Collection (called "ATCC" for short) and used (Registration No. ATCC HB8065).

It was confirmed by radioimmunoassay (called "RIA" for short) that the Hep G2 cell strain actually produced human $\alpha_2$-plasmin inhibitor. As a matter of fact, Rabbit anti-human $\alpha_2$-plasmin inhibitor antibody received from Behringwerke A.G., West Germany was labeled with $^{125}$I according to a conventional method, and an RIA was run on supernatant of a Hep G2 cell culture. On day 5 of the culture of Hep G2 cells a number of the cells reached $1.5 \times 10^7$ per culture dish (∅ 9 cm), that is, the confluent stage at which time there was contained in the supernatant $\alpha_2$-plasmin inhibitor at a count of 1380 cpm (background 0-16 cpm) and in an amount of 190 ng/ml. On the basis of the progress of the amount of $\alpha_2$-plasmin inhibitor in the supernatant from the Hep G2 cells, it was decided to collect on about day 2 after initiation of the culture the Hep G2 cells expected to contain the largest amount of a messenger RNA coding said protein, that is, when number of the cells was $4 \times 10^6$ per culture dish (early log stage). Namely, $4 \times 10^6$ cell/culture dish x 40 dishes, i.e. $1 \times 10^8$ total number of the cells were employed as RNA sources. In order to collect the cells on the culture dishes, the culture fluid was removed by suction, and the cells sticked to the bottom was suspended in GTC solution (an aqueous solution composed of 6M guanidinium isothiocyanate, 5 mM sodium citrate, 0.5% sarcosine and 0.1M $\beta$-mercaptoethanol) and stripped off. The suspension of the cells in GTC was passed three times through a gauge-18 syringe to destroy the cells. The suspension of the destroyed cells (7 ml) was overlayed on 3 ml of a 5.7 M CsCl-0.1M EDTA solution, and the mixture was centrifuged at 35,000 rev./min. at 20°C for 20 hours. The RNA fractions of a higher density were collected at the bottom of the centrifugal tube as pellets. The RNA pellet was washed once with 8M-guanidine-10 mM sodium acetate (pH 5.2)-1 mM dithiothreitol (DTT) solution and once with a 75% ethanol solution and finally stored in a 75% ethanol solution containing 0.3M sodium acetate (pH 5.2) at -80°C. The yield was 2.8 mg in terms of the RNA from $1.6 \times 10^8$ cells. The guanidinium isothiocyanate/cesium chloride method employed above was carried out in accordance with "Molecular Cloning-A Laboratory Manual" (edited by Maniatis, T. et al. (1982); published by Cold Spring Harbor Laboratory), p. 196 and the following. 50 $\mu$g of poly A RNA was thus obtained from the total RNA by oligo dT-cellulose column chromatography (manufactured by Sigma) according to a conventional method.

Example 2

Preparation of Hep G2 library

A cDNA library was prepared from the poly A messenger RNA derived from Hep G2 cells obtained in Example 1 by the method of Watson et al. (Reference: Glover, D.M. ed. "DNA cloning, vol. 1" (1985), published by IRL Press, Oxford/Washington D.C.; pp. 79-88).

In 10 $\mu$l of $H_2O$ was dissolved 10 $\mu$g of the poly A messenger RNA derived from Hep G2. The solution was heated at 65°C for 5 min. and rapidly cooled in ice to denature RNA. The RNA solution was prepared at a final concentration of 50 mM Tris HCl (pH 8.3), 8 mM $MgCl_2$ and 50 mM KCl, 4 mM each of dATP, dCTP, dGTP and dTTP and 10 mM of dithiothreitol (DTT), and 60 units of a ribonuclease inhibitor RNasin (availbale from Takara Shuzo), 2 $\mu$g of oligo $(dT)_{12-18}$ (available from Pharmacia) as a primer, 10 $\mu$Ci of $\alpha^{32}p$-dCTP (available from Amersham) as a tracer and 100 units of a reverse transcriptase (available from Takara Shuzo) to a final liquid volume of 100 $\mu$l. The solution was reacted at 42°C for 2 hours to synthesize DNA which was complementary to mRNA (first strand complementary DNA). The reaction was terminated by successively adding to the reaction mixture 5 $\mu$l of 0.5M EDTA (pH 8.0), 2.5 $\mu$l of 10% SDS (sodium dodecylsulfate), 50 $\mu$l of phenol and 50 $\mu$l of chloroform. To the reaction mixture (aqueous layer) were added an equal volume of 4M ammonium acetate and a four volumes of ethanol to precipitate cDNA-RNA hybrids. Reprecipitation of the hybrids by ethanol was repeated following dissolution in TE solution (10 mM Tris-HCl, pH 8.0, 1 mM EDTA). Precipitates of the cDNA-RNA hybrids were dissolved in 30 $\mu$l of TE, and then the RNA strand of the cDNA-RNA hybrid was replaced by a second strand cDNA in such a way as described below. A solution of the cDNA-RNA hybrid were prepared at a final concentrations of 100 mM HEPES (pH 7.6), 4 mM $MgCl_2$, 1.5 mM $\beta$-mercaptoethanol and 67.5 mM KCl and 0.5 mM each of dATP, dCTP, dGTP and dTTP, 0.15 mM $\beta$-nicotinamide adenine dinucleotide ($\beta$-NAD) and 10 $\mu$Ci of $\alpha^{32}p$-dCTP as a tracer and 150 units of E. coli DNA polymerase I (available from Takara Shuzo) and 5 units of RNase II (available from Takara Shuzo) to a final liquid volume of 100 $\mu$l. The resulting mixture was reacted at 14°C for 1 hour and further at room temperature for 1 hour. Termination of the reaction was performed in the same way as in the synthesis of the first-strand cDNA, and finally double-stranded cDNAs were precipitated by adding an equal volume of 4M ammonium acetate and four volumes of ethanol at -70°C. Both ends of the double-stranded DNAs were subjected to a filling reaction with T4 DNA polymerase (available from Takara Shuzo) in the presence of 0.7 mM each of dATP, dCTP, dGTP, dTTP to produce a blunt end. Methylation of the double-stranded cDNAs were performed with 5 units of EcoRI methylase (available from Bethesda Res. Lab.) to protect the EcoRI sites from EcoRI digestion, and then EcoRI linker (available fro Takara Shuzo) was added with 200 units of T4 DNA ligase (available from Takara Shuzo) to the two ends of the blunt-end double-stranded cDNA and finally the EcoRI linker was cut and removed with 200 units of the EcoRI. There was prepared in the series of reactions a double-stranded cDNA having EcoRI sites at the two ends. The double-stranded cDNAs were inserted into phage genomes by using a lambda gt11 cloning system (available from Seikagaku Kogyo under the trade name Protoclone™GT). The double-strand cDNA thus prepared with EcoRI sites at the two ends was ligated with a λgt11 vector digested with EcoRI by means of T4 DNA ligase. A packaging into phage was conducted by the use of a lambda DNA packaging system (trade name Packagene ®) to infect the host E. coli Y1090 (a strain defective in intracellular protease, available from Seikagaku Kogyo attached to Protoclone ™GT) to construct a cDNA library consisting of $2 \times 10^5$ phages.

Example 3

Screening by the synthetic probe

69mer Oligonucleotide probe was synthesized by an automatic DNA-synthesizer (Table 1) corresponding to a length of 23 amino acid residues in accordance with the partial amino acid sequence of $\alpha_2$-plasmin inhibitor published by Lijnen et al. (Thromb Haemostas (Stuttgart) 48 (1982), pp. 311-314, op. cit.) and used.

The synthetic probe, which was synthesized together with a complementary strand and designed in such a way that the two ends are EcoRI site and PstI site. It was inserted between the EcoRI site and the PstI site of the cloning vector M13-mp19 commercially available from Takara Shuzo. The single-stranded recombinant M13 DNA was prepared and $^{32}P$-labeled complementary strand was synthesize by primer extension using a synthetic primer (also available from Takara Shuzo) and a Klenow enzyme (Takara Shuzo) in the presence of $\alpha$-$^{32}$p-dCTP (Amersham, 3000 Ci/mmol). It was digested with the EcoRI and the PstI and subjected to electrophoresis with 10% polyacrylamide sequencing gel. The $^{32}$P-labeled probe DNA band detected by autoradiography was isolated and purified by electroelution (170V, overnight). Screening of the cDNA library by using the labeled synthetic probe was performed by plaque hybridization. The method is in accordance with a literature "Molecular Cloning-A Laboratory Manual" (op. cit.), p. 326 and the following. A nitrocellulose filter (∅ 82 mm) bound with the plaque was washed with a prewashing solution (50 mM Tris-HC1 (pH 8.0), 1 M NaCl, 1 mM EDTA, 0.1% SDS) at 42°C for 1 hour. Subsequently, 4 sheets of the nitrocellulose filter were placed per vinyl bag, to which was added 12 ml per bag of a prehibridization solution (50% formamide, 5 x Denhardt's solution, 5 x SSPE, 0.1% SDS, 100 $\mu$g/ml degraded salmon sperm DNA). The bags were heat-sealed and the DNA filters were incubated at 42°C. The prehybridization solution was removed from the bag, to which were added 10 ml per bag of fresh prehybridization solution and 300 $\mu$l of the $^{32}$P-labeled synthetic DNA probe solution after heat-denaturation at 100°C for 5 min. and rapidly cooled. A hybridization was carried out at 42°C for 2 nights. The nitrocellulose filters after the hybridization were washed four times with 6 x SSC solution at room temperature for 10 min. and twice with 2 x SSC/0.1% SDS solution at 42°C for 30 min. and air dried on a filter. Radioautography was performed using an X-ray film (available from Fuji Film) overnight at -70°C.

There were thus obtained 34 clones with moderate to strong signal. By determining the partial DNA sequence, one clone containing a 670bp cDNA fragment which codes $\alpha_2$-plasmin inhibitor (called $\lambda$ APH34) was obtained. Concurrently, a similar screening was carried out with a human liver cDNA library available from Toyo Boseki, thereby obtaining 8 clones with the same synthetic probe. It seemed that the 8 clones would be sister clones, on the basis of restriction analysis. One representative of these was called $\lambda$ APL1.

Example 4

Preparation of cDNA with the entire length of $\alpha_2$-plasmin inhibitor

The EcoRI insert fragment of $\lambda$ APH34 obtained from the Hep G2 cDNA library and the EcoRI insert fragment (1570 bp in length) of $\lambda$ APL1 obtained from the human liver cDNA library were fused to prepare APHL which encodes the entire $\alpha_2$-plasmin inhibitor-like protein. The APHL fragment was inserted into EcoRI site of the plasmid vector pHSG 299 (presented by Sato et al. of Hoechst Japan Ltd. at the 1985 Annual Conference of the Molecular Biological Society of Japan (December 2-5, 1985) which has kanamycin resistance derived from transposon Tn903 (Reference: Oka, A. et al.: J. Mol. Biol. (1981), 148, 217-226) and a polylinker derived from pUC19 (available from Takara Shuzo), and the resulting plasmid was designated p$\alpha$AP3. Host E. coli K12/Om 214 was transcribed with the p$\alpha$AP3. The transformed E. coli with the p$\alpha$AP3 containing APHL was deposited at Fermentation Research Institute, Agency of Industrial Science and Technology as FERM BP-1350. Restriction enzyme map of the APHL is shown in the attached drawing.

Example 5

Determination of the base sequence of $\alpha_2$-plasmin inhibitor cDNA(APHL)

As APHL has restriction enzyme sites as shown in the attached drawing, a subcloning was done at these sites in the polylinker region of M13 vector and the inserted DNA sequences were determined by dideoxy method (Reference: Sanger, F. et al.: Science (1981) 214: 1205-1210) using M13 sequencing kit from Takara Shuzo (Table 2). The comparison with the known peptide chain of human $\alpha_2$-plasmin inhibitor as described in literatures is as follows:

Known peptide chain A (Reference 3)

    NH₂-Asn-Gln-Glu-Gln-Val-Ser-Pro-Leu-Thr-
                                *
    Gly-Leu-Lys.

(Corresponding to the peptide coded with the bases 142-177 in Table 2. The glycin in the peptide chain A replaced by leucine)

Known peptide chain B (Reference 1)

    Leu-Gly-Asn-Gln-Glu-Pro-Gly-Gly-Gln-Thr-

    Ala-Leu-Lys-Ser-Pro-Pro-Gly-Val-Cys-Ser-

    Arg

(Corresponding to the peptide coded with the bases 178-240 in Table 2)

Known peptide chain C (Reference 1)

    Arg-Leu-Ala-Ala-Arg-Met-Tyr-Leu-Gln-Lys-

    Gly-Phe-Pro-Ile-Lys-Glu-Asp-Phe-Leu-Glu-
                                *
    Gln-Ser-Leu

(Corresponding to the peptide coded with the bases 505-573 in Table 2. The leucine marked * in the peptide chain C replaced by glutamic acid.)

Known peptide chain D (Reference 1)

    Pro-Val-Ser-Leu-Thr-Gly-Lys-Gln-Glu-Asp-

    Asp-Leu-Ala-Asn-Ile-Asn-Gln-Trp-Val-Lys-

    Glu-Ala-Thr-Glu-Gly-Lys-Ile-Gln-Glu-Phe

(Corresponding to the peptide coded with the bases 591-681 in Table 2)

Known peptide chain E (Reference 1)

```
        *         *
Ser-Leu-Lys-Phe-Asp-Pro-Ser-Leu-Thr-Gln-
                      *     *
Arg-Asp-Ser-Phe-Leu-His-Asp-Glu-Gln-Phe-

Thr-Val-Pro-Val-Glu-Met-Met-Gln-Ala-Arg-
 *
Val-Tyr-Pro
```

(Corresponding to the peptide coded with the bases 748-846 in Table 2. The amino acids marked * in the peptide chain E replaced by arginine, asparagine, histidine, leucine and threonine, respectively.)

Known peptide chain F (Reference 4)

```
Met-Ser-Phe-Val-Val Leu-Val
```

(Corresponding to the peptide coded with the bases 909-931 in Table 2)

Known peptide chain G (Reference 2)

```
Phe-Val-Gly-Ser-Val-Arg-Asn-Pro-Asn-Pro-

Ser-Ala-Pro-Arg-Glu-Leu-Lys-Glu-Gln-Gln-

Asp-Ser-Pro-Gly-Asn-Lys
```

(Corresponding to the peptide coded with the bases 1308-1386 in Table 2)

Known peptide H (Reference 1)

```
Lys-Gly-Phe-Pro-Arg-Gly-Asp-Lys-Leu-Phe-

Gly-Pro-Asp-Leu-Lys-Leu-Val-Pro-Pro-Met-

Glu-Glu-Asp-Tyr-Pro-Gln-Phe-Gly-Ser-Pro-

Lys
```

(Corresponding to the peptide coded with the bases 1405-1497 in Table 2)

17

Reference 1

H.R. Lijnen et al., Thromb Haemostas (Stuttgart) $\underline{48}$ (3) 311-314 (1982)

Reference 2

H.R. Lijnen et al., Thrombosis Research $\underline{27}$ (1) 83-89 (1982)

Reference 3

Akitada Ichinose et al., Febs Letters $\underline{153}$ (2) 369-371 (1983)

Reference 4

B. Wiman, Methods in Enzymology $\underline{80}$ 395-408 (1981)

**Claims**

1) DNA coding human $\alpha_2$-plasmin inhibitor-like protein of the amino acid sequence,

1)  Asn-Gln-Glu-Gln-Val-Ser-Pro-Leu-Thr-
Leu-Leu-Lys-Leu-Gly-Asn-Gln-Glu-Pro-Gly-
Gly-Gln-Thr-Ala-Leu-Lys-Ser-Pro-Pro-Gly-
Val-Cys-Ser-Arg-Asp-Pro-Thr-Pro-Glu-Gln-
Thr-His-Arg-Leu-Ala-Arg-Ala-Met-Met-Ala-
Phe-Thr-Ala-Asp-Leu-Phe-Ser-Leu-Val-Ala-
Gln-Thr-Ser-Thr-Cys-Pro-Asn-Leu-Ile-Leu-
Ser-Pro-Leu-Ser-Val-Ala-Leu-Ala-Leu-Ser-
His-Leu-Ala-Leu-Gly-Ala-Gln-Asn-His-Thr-
Leu-Gln-Arg-Leu-Gln-Gln-Val-Leu-His-Ala-
Gly-Ser-Gly-Pro-Cys-Leu-Pro-His-Leu-Leu-
Ser-Arg-Leu-Cys-Gln-Asp-Leu-Gly-Pro-Gly-
Ala-Phe-Arg-Leu-Ala-Ala-Arg-Met-Tyr-Leu-
Gln-Lys-Gly-Phe-Pro-Ile-Lys-Glu-Asp-Phe-
Leu-Glu-Gln-Ser-Glu-Gln-Leu-Phe-Gly-Ala-
Lys-Pro-Val-Ser-Leu-Thr-Gly-Lys-Gln-Glu-
Asp-Asp-Leu-Ala-Asn-Ile-Asn-Gln-Trp-Val-

Lys-Glu-Ala-Thr-Glu-Gly-Lys-Ile-Gln-Glu-

Phe-Leu-Ser-Gly-Leu-Pro-Glu-Asp-Thr-Val-

Leu-Leu-Leu-Leu-Asn-Ala-Ile-His-Phe-Gln-

Gly-Phe-Trp-Arg-Asn-Lys-Phe-Asp-Pro-Ser-

Leu-Thr-Gln-Arg-Asp-Ser-Phe-His-Leu-Asp-

Glu-Gln-Phe-Thr-Val-Pro-Val-Glu-Met-Met-

Gln-Ala-Arg-Thr-Tyr-Pro-Leu-Arg-Trp-Phe-

Leu-Leu-Glu-Gln-Pro-Glu-Ile-Gln-Val-Ala-

His-Phe-Pro-Phe-Lys-Asn-Asn-Met-Ser-Phe-

Val-Val-Leu-Val-Pro-Thr-His-Phe-Glu-Trp-

Asn-Val-Ser-Gln-Val-Leu-Ala-Asn-Leu-Ser-

Trp-Asp-Thr-Leu-His-Pro-Pro-Leu-Val-Trp-

Glu-Arg-Pro-Thr-Lys-Val-Arg-Leu-Pro-Lys-

Leu-Tyr-Leu-Lys-His-Gln-Met-Asp-Leu-Val-

Ala-Thr-Leu-Ser-Gln-Leu-Gly-Leu-Gln-Glu-

Leu-Phe-Gln-Ala-Pro-Asp-Leu-Arg-Gly-Ile-

Ser-Glu-Gln-Ser-Leu-Val-Val-Ser-Gly-Val-

Gln-His-Gln-Ser-Thr-Leu-Glu-Leu-Ser-Glu-

Val-Gly-Val-Glu-Ala-Ala-Ala-Ala-Thr-Ser-

Ile-Ala-Met-Ser-Arg-Met-Ser-Leu-Ser-Ser-

Phe-Ser-Val-Asn-Arg-Pro-Phe-Leu-Phe-Phe-

Ile-Phe-Glu-Asp-Thr-Thr-Gly-Leu-Pro-Leu-

Phe-Val-Gly-Ser-Val-Arg-Asn-Pro-Asn-Pro-

Ser-Ala-Pro-Arg-Glu-Leu-Lys-Glu-Gln-Gln-

Asp-Ser-Pro-Gly-Asn-Lys-Asp-Phe-Leu-Gln-

Ser-Leu-Lys-Gly-Phe-Pro-Arg-Gly-Asp-Lys-

Leu-Phe-Gly-Pro-Asp-Leu-Lys-Leu-Val-Pro-

Pro-Met-Glu-Glu-Asp-Tyr-Pro-Gln-Phe-Gly-

Ser-Pro-Lys .

2) DNA according to claim 1 having the base sequence,

AAC.CAG.GAG.CAG.GTG.TCC.CCA.CTT.ACC.

CTC.CTC.AAG.TTG.GGC.AAC.CAG.GAG.CCT.GGT.

GGC.CAG.ACT.GCC.CTG.AAG.AGT.CCC.CCA.GGA.

GTC.TGC.AGC.AGA.GAC.CCC.ACC.CCA.GAG.CAG.

ACC.CAC.AGG.CTG.GCC.CGG.GCC.ATG.ATG.GCC.

TTC.ACT.GCC.GAC.CTG.TTC.TCC.CTG.GTG.GCT.

CAA.ACG.TCC.ACC.TGC.CCC.AAC.CTC.ATC.CTG.

TCA.CCC.CTG.AGT.GTG.GCC.CTG.GCG.CTG.TCT.

CAC.CTG.GCA.CTA.GGT.GCT.CAG.AAC.CAC.ACG.

TTG.CAG.AGG.CTG.CAA.CAG.GTG.CTG.CAC.GCA.

```
GGC.TCA.GGG.CCC.TGC.CTC.CCC.CAT.CTG.CTG.

AGC.CGC.CTC.TGC.CAG.GAC.CTG.GGC.CCC.GGC.

GCG.TTC.CGA.CTG.GCT.GCC.AGG.ATG.TAC.CTG.

CAG.AAA.GGA.TTT.CCC.ATC.AAA.GAA.GAT.TTC.

CTG.GAA.CAA.TCC.GAA.CAG.CTA.TTT.GGG.GCA.

AAG.CCC.GTG.AGC.CTG.ACG.GGA.AAG.CAG.GAA.

GAT.GAC.CTG.GCA.AAC.ATC.AAC.CAA.TGG.GTG.

AAG.GAG.GCC.ACG.GAG.GGG.AAG.ATT.CAG.GAA.

TTC.CTC.TCT.GGG.CTG.CCG.GAA.GAC.ACC.GTG.

TTG.CTT.CTC.CTC.AAC.GCC.ATC.CAC.TTC.CAG.

GGT.TTC.TGG.AGG.AAC.AAG.TTT.GAC.CCG.AGC.

CTT.ACC.CAG.AGA.GAC.TCC.TTC.CAC.CTG.GAC.

GAG.CAG.TTC.ACG.GTG.CCC.GTG.GAA.ATG.ATG.

CAG.GCC.CGC.ACG.TAC.CCG.CTG.CGC.TGG.TTC.

TTG.CTG.GAG.CAG.CCT.GAG.ATC.CAG.GTG.GCT.

CAT.TTC.CCC.TTT.AAG.AAC.AAC.ATG.AGC.TTT.

GTG.GTC.CTT.GTA.CCC.ACC.CAC.TTT.GAA.TGG.

AAC.GTG.TCC.CAG.GTA.CTG.GCC.AAC.CTG.AGT.

TGG.GAC.ACC.CTG.CAC.CCA.CCT.CTG.GTG.TGG.
```

```
GAG.AGG.CCC.ACC.AAG.GTC.CGG.CTG.CCT.AAG.

CTG.TAT.CTG.AAA.CAC.CAA.ATG.GAC.CTG.GTG.

GCC.ACC.CTC.AGC.CAG.CTG.GGC.CTG.CAG.GAG.

TTG.TTC.CAG.GCC.CCA.GAC.CTG.CGT.GGG.ATC.

TCC.GAG.CAG.AGC.CTG.GTG.GTG.TCC.GGC.GTG.

CAG.CAT.CAG.TCC.ACC.CTG.GAG.CTC.AGC.GAG.

GTC.GGC.GTG.GAG.GCG.GCG.GCG.GCC.ACC.AGC.

ATT.GCC.ATG.TCC.CGC.ATG.TCC.CTG.TCC.TCC.

TTC.AGC.GTG.AAC.CGC.CCC.TTC.CTC.TTC.TTC.

ATC.TTC.GAG.GAC.ACC.ACA.GGC.CTT.CCC.CTC.

TTC.GTG.GGC.AGC.GTG.AGG.AAC.CCC.AAC.CCC.

AGT.GCA.CCG.CGG.GAG.CTC.AAG.GAA.CAG.CAG.

GAT.TCC.CCG.GGC.AAC.AAG.GAC.TTC.CTC.CAG.

AGC.CTG.AAA.GGC.TTC.CCC.CGC.GGA.GAC.AAG.

CTT.TTC.GGC.CCT.GAC.TTA.AAA.CTT.GTG.CCC.

CCC.ATG.GAG.GAG.GAT.TAC.CCC.CAG.TTT.GGC.

AGC.CCC.AAG.
```

3. Human α₂-plasmin inhibitor-like protein having the amino acid sequence given in claim 1.

4. A process for making a human α₂-plasmin inhibitor-like protein of the amino acid sequence given in claim 1 which comprises expressing in a host cell a DNA coding for said protein.

5. A process as claimed in claim 4, wherein the DNA has the base sequence given in claim 2.

6. A process as claimed in claim 4 or 5, wherein the host cell is a mammalian cell.

Claims for the following contracting states: AT, ES, GR

1. A process for making a human α₂-plasmin inhibitor-like protein of the amino acid sequence

1)  Asn-Gln-Glu-Gln-Val-Ser-Pro-Leu-Thr-

Leu-Leu-Lys-Leu-Gly-Asn-Gln-Glu-Pro-Gly-

Gly-Gln-Thr-Ala-Leu-Lys-Ser-Pro-Pro-Gly-

Val-Cys-Ser-Arg-Asp-Pro-Thr-Pro-Glu-Gln-

Thr-His-Arg-Leu-Ala-Arg-Ala-Met-Met-Ala-

Phe-Thr-Ala-Asp-Leu-Phe-Ser-Leu-Val-Ala-

Gln-Thr-Ser-Thr-Cys-Pro-Asn-Leu-Ile-Leu-

Ser-Pro-Leu-Ser-Val-Ala-Leu-Ala-Leu-Ser-

His-Leu-Ala-Leu-Gly-Ala-Gln-Asn-His-Thr-

Leu-Gln-Arg-Leu-Gln-Gln-Val-Leu-His-Ala-

Gly-Ser-Gly-Pro-Cys-Leu-Pro-His-Leu-Leu-

Ser-Arg-Leu-Cys-Gln-Asp-Leu-Gly-Pro-Gly-

Ala-Phe-Arg-Leu-Ala-Ala-Arg-Met-Tyr-Leu-

Gln-Lys-Gly-Phe-Pro-Ile-Lys-Glu-Asp-Phe-

Leu-Glu-Gln-Ser-Glu-Gln-Leu-Phe-Gly-Ala-

Lys-Pro-Val-Ser-Leu-Thr-Gly-Lys-Gln-Glu-

Asp-Asp-Leu-Ala-Asn-Ile-Asn-Gln-Trp-Val-

Lys-Glu-Ala-Thr-Glu-Gly-Lys-Ile-Gln-Glu-

Phe-Leu-Ser-Gly-Leu-Pro-Glu-Asp-Thr-Val-

Leu-Leu-Leu-Leu-Asn-Ala-Ile-His-Phe-Gln-

Gly-Phe-Trp-Arg-Asn-Lys-Phe-Asp-Pro-Ser-

Leu-Thr-Gln-Arg-Asp-Ser-Phe-His-Leu-Asp-

Glu-Gln-Phe-Thr-Val-Pro-Val-Glu-Met-Met-

Gln-Ala-Arg-Thr-Tyr-Pro-Leu-Arg-Trp-Phe-

Leu-Leu-Glu-Gln-Pro-Glu-Ile-Gln-Val-Ala-

His-Phe-Pro-Phe-Lys-Asn-Asn-Met-Ser-Phe-

Val-Val-Leu-Val-Pro-Thr-His-Phe-Glu-Trp-

Asn-Val-Ser-Gln-Val-Leu-Ala-Asn-Leu-Ser-

Trp-Asp-Thr-Leu-His-Pro-Pro-Leu-Val-Trp-

Glu-Arg-Pro-Thr-Lys-Val-Arg-Leu-Pro-Lys-

Leu-Tyr-Leu-Lys-His-Gln-Met-Asp-Leu-Val-

Ala-Thr-Leu-Ser-Gln-Leu-Gly-Leu-Gln-Glu-

Leu-Phe-Gln-Ala-Pro-Asp-Leu-Arg-Gly-Ile-

Ser-Glu-Gln-Ser-Leu-Val-Val-Ser-Gly-Val-

Gln-His-Gln-Ser-Thr-Leu-Glu-Leu-Ser-Glu-

Val-Gly-Val-Glu-Ala-Ala-Ala-Ala-Thr-Ser-

Ile-Ala-Met-Ser-Arg-Met-Ser-Leu-Ser-Ser-

Phe-Ser-Val-Asn-Arg-Pro-Phe-Leu-Phe-Phe-

```
Ile-Phe-Glu-Asp-Thr-Thr-Gly-Leu-Pro-Leu-
Phe-Val-Gly-Ser-Val-Arg-Asn-Pro-Asn-Pro-
Ser-Ala-Pro-Arg-Glu-Leu-Lys-Glu-Gln-Gln-
Asp-Ser-Pro-Gly-Asn-Lys-Asp-Phe-Leu-Gln-
Ser-Leu-Lys-Gly-Phe-Pro-Arg-Gly-Asp-Lys-
Leu-Phe-Gly-Pro-Asp-Leu-Lys-Leu-Val-Pro-
Pro-Met-Glu-Glu-Asp-Tyr-Pro-Gln-Phe-Gly-
Ser-Pro-Lys ,
```

which comprises expressing in a host cell a DNA coding for said protein.

2. A process as claimed in claim 1, wherein the DNA has the base sequence

```
AAC.CAG.GAG.CAG.GTG.TCC.CCA.CTT.ACC.
CTC.CTC.AAG.TTG.GGC.AAC.CAG.GAG.CCT.GGT.
GGC.CAG.ACT.GCC.CTG.AAG.AGT.CCC.CCA.GGA.
GTC.TGC.AGC.AGA.GAC.CCC.ACC.CCA.GAG.CAG.
ACC.CAC.AGG.CTG.GCC.CGG.GCC.ATG.ATG.GCC.
TTC.ACT.GCC.GAC.CTG.TTC.TCC.CTG.GTG.GCT.
CAA.ACG.TCC.ACC.TGC.CCC.AAC.CTC.ATC.CTG.
TCA.CCC.CTG.AGT.GTG.GCC.CTG.GCG.CTG.TCT.
CAC.CTG.GCA.CTA.GGT.GCT.CAG.AAC.CAC.ACG.
TTG.CAG.AGG.CTG.CAA.CAG.GTG.CTG.CAC.GCA.
```

```
GGC.TCA.GGG.CCC.TGC.CTC.CCC.CAT.CTG.CTG.

AGC.CGC.CTC.TGC.CAG.GAC.CTG.GGC.CCC.GGC.

GCG.TTC.CGA.CTG.GCT.GCC.AGG.ATG.TAC.CTG.

CAG.AAA.GGA.TTT.CCC.ATC.AAA.GAA.GAT.TTC.

CTG.GAA.CAA.TCC.GAA.CAG.CTA.TTT.GGG.GCA.

AAG.CCC.GTG.AGC.CTG.ACG.GGA.AAG.CAG.GAA.

GAT.GAC.CTG.GCA.AAC.ATC.AAC.CAA.TGG.GTG.

AAG.GAG.GCC.ACG.GAG.GGG.AAG.ATT.CAG.GAA.

TTC.CTC.TCT.GGG.CTG.CCG.GAA.GAC.ACC.GTG.

TTG.CTT.CTC.CTC.AAC.GCC.ATC.CAC.TTC.CAG.

GGT.TTC.TGG.AGG.AAC.AAG.TTT.GAC.CCG.AGC.

CTT.ACC.CAG.AGA.GAC.TCC.TTC.CAC.CTG.GAC.

GAG.CAG.TTC.ACG.GTG.CCC.GTG.GAA.ATG.ATG.

CAG.GCC.CGC.ACG.TAC.CCG.CTG.CGC.TGG.TTC.

TTG.CTG.GAG.CAG.CCT.GAG.ATC.CAG.GTG.GCT.

CAT.TTC.CCC.TTT.AAG.AAC.AAC.ATG.AGC.TTT.

GTG.GTC.CTT.GTA.CCC.ACC.CAC.TTT.GAA.TGG.

AAC.GTG.TCC.CAG.GTA.CTG.GCC.AAC.CTG.AGT.

TGG.GAC.ACC.CTG.CAC.CCA.CCT.CTG.GTG.TGG.
```

```
GAG.AGG.CCC.ACC.AAG.GTC.CGG.CTG.CCT.AAG.
CTG.TAT.CTG.AAA.CAC.CAA.ATG.GAC.CTG.GTG.
GCC.ACC.CTC.AGC.CAG.CTG.GGC.CTG.CAG.GAG.
TTG.TTC.CAG.GCC.CCA.GAC.CTG.CGT.GGG.ATC.
TCC.GAG.CAG.AGC.CTG.GTG.GTG.TCC.GGC.GTG.
CAG.CAT.CAG.TCC.ACC.CTG.GAG.CTC.AGC.GAG.
GTC.GGC.GTG.GAG.GCG.GCG.GCG.GCC.ACC.AGC.
ATT.GCC.ATG.TCC.CGC.ATG.TCC.CTG.TCC.TCC.
TTC.AGC.GTG.AAC.CGC.CCC.TTC.CTC.TTC.TTC.
ATC.TTC.GAG.GAC.ACC.ACA.GGC.CTT.CCC.CTC.
TTC.GTG.GGC.AGC.GTG.AGG.AAC.CCC.AAC.CCC.
AGT.GCA.CCG.CGG.GAG.CTC.AAG.GAA.CAG.CAG.
GAT.TCC.CCG.GGC.AAC.AAG.GAC.TTC.CTC.CAG.
AGC.CTG.AAA.GGC.TTC.CCC.CGC.GGA.GAC.AAG.
CTT.TTC.GGC.CCT.GAC.TTA.AAA.CTT.GTG.CCC.
CCC.ATG.GAG.GAG.GAT.TAC.CCC.CAG.TTT.GGC.
AGC.CCC.AAG.
```

3. A process as claimed in claim 1 or 2, wherein the host cell is a mammalian cell.

Pvull
EcoRI | Ncol   PstI          DNA Probe ▨▨

DNA Probe ▨▨            PstI      EcoRI
λAPH34

A B                    C   D

DNA Probe ▨▨

                                                    SacI
                              SmaI  HindIII        SmaI  SacI
                        Pvull  PstI  SacI          SmaI Xhol  EcoRI
λ APL1 ----            EcoRI            Ncol   Pvull

        C   D    E  F              G   H

                                                    SacI
Pvull                     SmaI  HindIII            SmaI  SacI
EcoRI | Ncol   PstI       PstI EcoRI    Pvull  PstI SacI   Ncol   Pvull SmaI Xhol  EcoRI
APHL

A B            C   D    E   F                  G   H

Portion coding α₂-plasmin inhibitor-like protein

100bp

A–H in the figure indicate known
peptide chains.

0 257 630

European Patent
Office

**EUROPEAN SEARCH REPORT**

Application number

| | DOCUMENTS CONSIDERED TO BE RELEVANT | | EP 87112381.6 |
|---|---|---|---|

| Category | Citation of document with indication, where appropriate, of relevant passages | Relevant to claim | CLASSIFICATION OF THE APPLICATION (Int. Cl 4) |
|---|---|---|---|
| D,A | THE JOURNAL OF LABORATORY AND CLINICAL MEDICINE, vol. 101, no. 3, 1983 (St. Louis) <br><br> D.S. FAIR et al. "Synthesis and secretion of the fibrinolytic components, including $\alpha_2$-antiplasmin, by a human hepatoma cell line" pages 372-384 <br><br>     * Totality * <br><br> -- | 1,3 | |
| A | PATENT ABSTRACTS OF JAPAN, unexamined applications, C field, vol. 7, no. 232, October 14, 1983 <br><br> THE PATENT OFFICE JAPANESE GOVERNMENT page 142 C 190 <br><br>     * Kokai-no. 58-124 798 (DAIICHI KAGAKU YAKUHIN K.K.) * <br><br> -- | 1,3 | |
| A | EP - A2 - 0 159 025 (TEIJIN LIMITED) <br><br>     * Pages 1,2 * <br><br> -- | 1,3 | TECHNICAL FIELDS SEARCHED (Int. Cl.4) |
| A | EP - A1 - 0 137 633 (ZYMOGENETICS, INC.) <br><br>     * Abstract * <br><br> -- | 1,2,4, 6 | |
| A | EP - A2 - 0 090 505 (GENENTECH, INC.) <br><br>     * Abstract * <br><br> ---- | 1,2,4 | |

The present search report has been drawn up for all claims

| Place of search | Date of completion of the search | Examiner |
|---|---|---|
| VIENNA | 03-12-1987 | WOLF |

European Patent
Office

**EUROPEAN SEARCH REPORT**

Application number

EP 87112381.6

| Category | Citation of document with indication, where appropriate, of relevant passages | Relevant to claim | CLASSIFICATION OF THE APPLICATION (Int. Cl.4) |
|---|---|---|---|
| D,A | FEBS LETTERS, vol. 153, no. 2, March 1983 (Amsterdam)<br><br>A. ICHINOSE et al. "Factor XIII-mediated cross-linking of $NH_2$-terminal peptide of $\alpha_2$-plasmin inhibitor to fibrin" pages 369-371<br><br>   * Totality *<br><br>   -- | 1,3 | C 12 N 15/00<br>C 07 H 21/04<br>C 12 P 21/00<br>C 07 K 13/00 |
| D,A | METHODS IN ENZYMOLOGY, vol. 80, 1981; Proteolytic Enzymes, Part C (New York)<br><br>B. WIMAN "Human $\alpha_2$-Antiplasmin" pages 395-408<br><br>   * Totality *<br><br>   -- | 1,3 | |
| D,A | THROMBOSIS AND HAEMOSTASIS, vol. 48, no. 3, 1982 (Stuttgart)<br><br>H.R. LIJNEN et al. "Partial Primary Structure of Human $\alpha_2$-Antiplasmin-Homology with Other Plasma Protease Inhibitors" pages 311-314<br><br>   * Totality *<br><br>   -- | 1,3 | **TECHNICAL FIELDS SEARCHED (Int. Cl.4)**<br><br>C 12 N<br>C 07 H<br>C 12 P<br>C 07 K |
| D,A | THROMBOSIS RESEARCH, vol. 27, no. 1, July 1, 1982 (New York)<br><br>H.R. LIJNEN et al. "Purification and Partial Primary Structure of Cyanogen Bromide Fragments from Human $\alpha_2$-Antiplasmin" pages 83-89<br><br>   -- | 1,3 | |

The present search report has been drawn up for all claims

| Place of search | Date of completion of the search | Examiner |
|---|---|---|
| VIENNA | 03-12-1987 | WOLF |